(19) Europäisches Patentamt / European Patent Office / Office européen des brevets

(11) **EP 4 159 210 A1**

(12) **EUROPEAN PATENT APPLICATION**
published in accordance with Art. 153(4) EPC

(43) Date of publication:
**05.04.2023 Bulletin 2023/14**

(21) Application number: **21813574.7**

(22) Date of filing: **28.05.2021**

(51) International Patent Classification (IPC):
*A61K 31/357* (2006.01)  *A61K 9/127* (2006.01)
*A61K 47/16* (2006.01)  *A61K 47/18* (2006.01)
*A61K 47/24* (2006.01)  *A61P 35/00* (2006.01)

(52) Cooperative Patent Classification (CPC):
**A61K 9/127; A61K 31/357; A61K 47/16;**
**A61K 47/18; A61K 47/24; A61P 35/00**

(86) International application number:
**PCT/CN2021/096944**

(87) International publication number:
**WO 2021/239134 (02.12.2021 Gazette 2021/48)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **29.05.2020  CN 202010475697**

(71) Applicants:
• **Jiangsu Hengrui Pharmaceuticals Co., Ltd.**
  **Lianyungang, Jiangsu 222047 (CN)**
• **Shanghai Hengrui Pharmaceutical Co., Ltd**
  **Shanghai 200245 (CN)**

(72) Inventors:
• **TONG, Xinyong**
  **Shanghai 200245 (CN)**

• **ZOU, Aifeng**
  **Shanghai 200245 (CN)**
• **ZHANG, Qibo**
  **Shanghai 200245 (CN)**
• **LIU, Dan**
  **Shanghai 200245 (CN)**
• **MA, Zhonghua**
  **Shanghai 200245 (CN)**
• **WANG, Yanping**
  **Shanghai 200245 (CN)**

(74) Representative: **dompatent von Kreisler Selting**
**Werner -**
**Partnerschaft von Patent- und Rechtsanwälten**
**mbB**
**Deichmannhaus am Dom**
**Bahnhofsvorplatz 1**
**50667 Köln (DE)**

(54) **LIPOSOME CONTAINING ETHYLENEDIAMINE TETRAACETIC ACID OR SALT THEREOF AND ERIBULIN OR PHARMACEUTICALLY ACCEPTABLE SALT THEREOF**

(57)    Provided are a liposome containing Eribulin or a pharmaceutically acceptable salt thereof and a preparation method therefor. An inner aqueous phase of the liposome contains Eribulin or a salt thereof, and one or more selected from ethylenediamine tetraacetic acid, phosphoric acid, pentetic acid, or salts thereof. Further provided are a pharmaceutical composition containing the aforementioned liposome and a preparation method therefor.

EP 4 159 210 A1

**Description**

The present application claims priority to Chinese Patent Application No. CN202010475697.3 filed on May 29, 2020, which is incorporated herein by reference in its entirety.

**TECHNICAL FIELD**

The present disclosure belongs to the field of pharmacy, and relates to a liposome containing ethylenediaminetetraacetic acid or a salt thereof and eribulin or a pharmaceutically acceptable salt thereof.

**BACKGROUND**

Eribulin is a synthetic analog of halichondrin B. It is a tubulin polymerization inhibitor with a novel mechanism of action, and was first approved by the U.S. FDA for the treatment of metastatic breast cancer in 2010.

However, after clinical intravenous administration, the drug in a free form is directly exposed to a slightly alkaline physiological environment and is prone to degradation, so that its activity is lost and the efficacy of the drug is indirectly reduced. Moreover, the formulation has relatively large toxic and side effects, mainly manifested by neutropenia and delayed diarrhea. As a drug carrier, the liposome has the characteristics of improving the efficacy of the drug, reducing the adverse reactions of the drug and providing targeting effects. However, it is not easy to truly achieve the therapeutic advantages of liposomes, and the following requirements must be met: 1) the drug can be encapsulated in liposomes with effective encapsulation efficiency and enough drug loading; 2) during the *in vitro* storage period, the drug will not be released from liposomes; 3) in the process of blood circulation, the drug in the liposome will not have significant leakage; 4) when liposomes are accumulated in the tumor area, the drug can be effectively released to exert its therapeutic effect.

WO2010113984 discloses a liposome composition containing eribulin, an ammonium salt and citric acid in an inner aqueous phase of a liposome to solve the problems concerning the stability and encapsulation efficiency of eribulin or a salt thereof in the liposome composition. Meanwhile, the examples show that the encapsulation efficiency of the liposome containing ammonium citrate or ammonium sulfate is better than that of liposomes comprising other ammonium salt systems such as ammonium acetate, ammonium phosphate or ammonium succinate. However, the long-term storage will cause the leakage of active ingredients and decrease the encapsulation efficiency of liposomes, which is not conducive to long-term storage of the samples.

In addition, CN105163720 discloses a method for preparing a liposome encapsulating a sparingly water-soluble agent, which comprises preparing a solution of the sparingly water-soluble agent by completely dissolving it in an aprotic solvent or polyol, and remotely loading an active ingredient, including carfilzomib, eribulin or the like, through a proton or ion gradient present across the liposome membrane. However, there is no actual preparation for the eribulin liposome in the examples, and eribulin has excellent water solubility. Therefore, the eribulin liposome having high encapsulation efficiency and stability cannot be obtained by the above method.

**SUMMARY**

The present disclosure provides a liposome, which contains eribulin or a pharmaceutically acceptable salt thereof as an active compound, and one or more selected from the group consisting of ethylenediaminetetraacetic acid, phosphoric acid, pentetic acid and salts thereof, in an inner aqueous phase.

In some embodiments, the ethylenediaminetetraacetate is a salt formed from ethylenediaminetetraacetic acid and an amine, a metal ion, or an ammonium ion. Examples of the ethylenediaminetetraacetate include, but are not limited to, ammonium ethylenediaminetetraacetate, sodium ethylenediaminetetraacetate, and calcium ethylenediaminetetraacetate.

In another aspect, the phosphate of the present disclosure is formed from phosphoric acid and an organic or inorganic base, and examples thereof include, but are not limited to, sodium phosphate, potassium phosphate, and ammonium phosphate.

In another aspect, the pentetate of the present disclosure is formed from pentetic acid and an organic or inorganic base, and examples thereof include, but are not limited to, sodium pentetate, potassium pentetate, and ammonium pentetate.

In some embodiments, the liposome further contains an ammonium salt, preferably ammonium sulfate, ethylammonium sulfate, ammonium ethylenediaminetetraacetate, ammonium chloride, ammonium hydroxide, ammonium acetate, ammonium phosphate, triethylammonium sucrose octasulfate, dextran ammonium sulfate, or triethyldextran ammonium sulfate, and more preferably ammonium sulfate, ammonium chloride, or ammonium ethylenediaminetetraacetate, in the inner aqueous phase.

In some embodiments, the liposome contains ammonium phosphate in the inner aqueous phase.

In some embodiments, the liposome contains eribulin or a pharmaceutically acceptable salt thereof as an active compound, ethylenediaminetetraacetic acid or a salt thereof, and an ammonium salt, in the inner aqueous phase.

In some embodiments, the liposome contains eribulin or a pharmaceutically acceptable salt thereof as an active compound, phosphoric acid or a salt thereof, and an ammonium salt, in the inner aqueous phase.

In some embodiments, the liposome contains eribulin or a pharmaceutically acceptable salt thereof as an active compound, pentetic acid or a salt thereof, and an ammonium salt, in the inner aqueous phase.

In some embodiments, the liposome further contains at least one pH adjuster in the inner aqueous phase, which may be selected from the group consisting of amino acids such as arginine, histidine or glycine; acids such as ethylenediaminetetraacetic acid, pentetic acid, hydrochloric acid, phosphoric acid or sulfuric acid; salts such as a sodium salt, a potassium salt, or an ammonium salt of the above acids; basic compounds (alkalis) such as ammonia water, sodium hydroxide or potassium hydroxide; and the like; preferably sodium hydroxide, hydrochloric acid, ammonia water, phosphoric acid, or ethylenediaminetetraacetic acid. In addition, the pH adjuster may be used in combination with two or more of the above ammonium salts.

In some embodiments, the ethylenediaminetetraacetic acid, phosphoric acid, pentetic acid, or salts thereof in the inner aqueous phase of the liposome has a concentration selected from the group consisting of 1-300 mM, which may be 1 mM, 2 mM, 3 mM, 4 mM, 5 mM, 6 mM, 7 mM, 8 mM, 9 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 155 mM, 160 mM, 165 mM, 170 mM, 175 mM, 180 mM, 185 mM, 190 mM, 195 mM, 200 mM, 205 mM, 210 mM, 215 mM, 220 mM, 225 mM, 230 mM, 235 mM, 240 mM, 245 mM, 250 mM, 255 mM, 260 mM, 265 mM, 270 mM, 275 mM, 280 mM, 285 mM, 290 mM, 295 mM, 300 mM or any value between any two of the values, preferably 20-200 mM.

In some other embodiments, the ammonium salt, e.g., ammonium sulfate, in the inner aqueous phase of the liposome has a concentration selected from the group consisting of 10-400 mM, which may be 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 155 mM, 160 mM, 165 mM, 170 mM, 175 mM, 180 mM, 185 mM, 190 mM, 195 mM, 200 mM, 205 mM, 210 mM, 215 mM, 220 mM, 225 mM, 230 mM, 235 mM, 240 mM, 245 mM, 250 mM, 255 mM, 260 mM, 265 mM, 270 mM, 275 mM, 280 mM, 285 mM, 290 mM, 295 mM, 300 mM or any value between any two of the values, preferably 20-200 mM.

The active compound of the present disclosure is eribulin or a pharmaceutically acceptable salt thereof. The pharmaceutically acceptable salt can be either an inorganic acid salt or an organic acid salt, and is not particularly limited as long as it forms a salt with eribulin, and examples thereof include hydrochloride, sulfate, hydrobromide, hydroiodide, nitrate, bisulfate, phosphate, perphosphate, isonicotinate, acetate, lactate, salicylate, tartrate, mesylate, ascorbate, succinate, maleate, fumarate, gluconate, saccharinate, formate, and benzoate, preferably hydrochloride, sulfate, acetate, phosphate, mesylate, and more preferably mesylate. In some embodiments, the active compound is eribulin mesylate.

In addition, the pharmacologically acceptable salt of eribulin may be a salt formed from eribulin and aluminum, calcium, lithium, magnesium, calcium, sodium, zinc or diethanolamine.

In some embodiments, the active compound has a concentration selected from the group consisting of 0.01-100 mg/mL, which may be 0.01 mg/mL, 0.05 mg/mL, 0.10 mg/mL, 0.15 mg/mL, 0.20 mg/mL, 0.25 mg/mL, 0.30 mg/mL, 0.35 mg/mL, 0.40 mg/mL, 0.45 mg/mL, 0.50 mg/mL, 0.55 mg/mL, 0.60 mg/mL, 0.65 mg/mL, 0.70 mg/mL, 0.75 mg/mL, 0.80 mg/mL, 0.85 mg/mL, 0.90 mg/mL, 0.95 mg/mL, 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL or any value between any two of the values, preferably 0.05-5 mg/mL.

In another aspect, the liposome of the present disclosure is substantially free of citric acid in the inner aqueous phase. The "substantially free of citric acid" means that no citric acid is added. It should be noted that it is sufficient to determine that, for example, the stability of the encapsulation efficiency of the liposome is not due to the addition of citric acid.

In some embodiments, the liposome contains eribulin or a pharmaceutically acceptable salt thereof as an active compound, one or more selected from the group consisting of ethylenediaminetetraacetic acid, phosphoric acid, pentetic acid and salts thereof, and an ammonium salt, in the inner aqueous phase.

In some embodiments, the liposome contains eribulin or a pharmaceutically acceptable salt thereof as an active compound, one or more selected from the group consisting of ethylenediaminetetraacetic acid, phosphoric acid, pentetic acid and salts thereof, and ammonium sulfate, in the inner aqueous phase.

In some embodiments, the liposome contains eribulin or a pharmaceutically acceptable salt thereof as an active compound, ethylenediaminetetraacetic acid, and ammonium ethylenediaminetetraacetate, in the inner aqueous phase.

In some embodiments, the liposome contains eribulin or a pharmaceutically acceptable salt thereof as an active compound, phosphoric acid, and ammonium phosphate, in the inner aqueous phase.

In another aspect, the liposome of the present disclosure further contains a phospholipid selected from, but not limited to, at least one of dipalmitoyl phosphatidylcholine (DPPC), distearoyl phosphatidylcholine (DSPC), dimyristoyl phosphatidylcholine (DMPC), 1-palmitoyl-2-linoleoyl-sn-glycero-3-phosphatidylcholine (PLPC), 1,2-dioleoyl-sn-glycero-3-phosphocholine (DOPC), egg yolk phosphatidylcholine (EPC), dilauroyl phosphatidylcholine (DLPC), hydrogenated egg phosphatidylcholine (HEPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoylphosphatidylcholine (SPPC), palmitoyl oleoylphosphatidylcholine (POPC), lysophosphatidylcholine, dilinoleoyl phosphatidylcholine, distearoyl phosphatidylethanolamine (DSPE), dimyristoyl phosphatidylethanolamine (DMPE), dipalmitoyl phosphatidylethanolamine (DPPE), dioleoyl phosphatidylglycerol (DOPG), dimyristoyl phosphatidylglycerol (DMPG), distearoyl phosphatidylglycerol (DSPG), dipalmitoyl glycerophosphoglycerol (DPPG), dipalmitoyl phosphatidylserine (DPPS), 1,2-dioleoyl-sn-glycero-3-phosphatidylserine (DOPS), dimyristoyl phosphatidylserine (DMPS), distearoyl phosphatidylserine (DSPS), 1,2-dipalmitoyl-sn-glycero-3-phosphatidic acid (DPPA), 1,2-dioleoyl-sn-glycero-3-phosphatidic acid (DOPA), 1,2-dimyristoyl-sn-glycero-3-phosphatidic acid (DMPA), 1,2-distearoyl-sn-glycero-3-phosphatidic acid (DSPA), dipalmitoyl phosphatidylinositol (DPPI), 1,2-dioleoyl-sn-glycero-3-phosphatidylinositol (DOPI), dimyristoyl phosphatidylinositol (DMPI), and distearoyl phosphatidylinositol (DSPI).

In some embodiments, the phospholipid is selected from one or more of hydrogenated egg phosphatidylcholine, distearoyl phosphatidylcholine, dipalmitoyl phosphatidylcholine, distearoyl phosphatidylcholine, 1,2-dioleoyl-sn-glycero-3-phosphocholine, and dimyristoyl phosphatidylcholine, wherein the hydrogenated egg phosphatidylcholine is preferably hydrogenated soy phosphatidylcholine.

In some embodiments, the phospholipid in the liposome has a content (relative to total liposome membrane components, mole fraction) is selected from the group consisting of 10%-80%, including but not limited to 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% or any value between any two of the values, preferably 30%-70%.

In another aspect, in some embodiments, the liposome further contains a steroid, including but not limited to cholesterol (CHOL).

In some embodiments, the steroid in the liposome has a content (relative to total liposome membrane components, mole fraction) selected from the group consisting of 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60% and any value between any two of the values, preferably 10%-40%.

In some embodiments, the liposome contains a phospholipid and a steroid such as cholesterol. In some embodiments, the liposome of the present disclosure contains 10%-80% phospholipid and 1%-60% steroid such as cholesterol.

In some embodiments, the liposome contains a phospholipid, cholesterol, and a distearoyl phosphatidylethanolamine-polyethylene glycol condensate, and contains eribulin or a pharmaceutically acceptable salt thereof as an active compound, one or more selected from the group consisting of ethylenediaminetetraacetic acid, phosphoric acid, pentetic acid and salts thereof, and an ammonium salt, in the inner aqueous phase.

The addition of one or more hydrophilic polymeric lipid derivatives to the liposome membrane material can be used to improve the number of cycles of the liposome-encapsulated active compound and avoid or reduce premature leakage of the active compound from the liposome composition. The attachment of PEG to the liposome is referred to as PEGylation. In exemplary embodiments of the present disclosure, the liposome is a PEGylated liposome. The hydrophilic polymer-modified lipids include, but are not limited to polyethylene glycol-modified distearoyl phosphatidylethanolamine (PEG-DSPE), polyethylene glycol-modified distearoyl phosphatidylglycerol (PEG-DSPG), and polyethylene glycol-modified cholesterol (PEG-CHOL). PEG has a molecular weight of 200-10,000 Da, preferably 1000-5000 Da, and more preferably 2000-5000 Da.

In some embodiments, the liposome further contains a hydrophilic polymer-modified lipid selected from one or more of polyethylene glycol-modified distearoyl phosphatidylethanolamine (PEG-DSPE), polyethylene glycol-modified distearoyl phosphatidylglycerol (PEG-DSPG), and polyethylene glycol-modified cholesterol (PEG-CHOL).

In some embodiments, the liposome contains polyethylene glycol-modified distearoyl phosphatidylethanolamine. In some embodiments, the polyethylene glycol in the polyethylene glycol-modified distearoyl phosphatidylethanolamine has a molecular weight of 200-10,000 Da. In some embodiments, the polyethylene glycol in the polyethylene glycol-modified distearoyl phosphatidylethanolamine has a molecular weight of 1000-5000 Da. In some embodiments, the polyethylene glycol in the polyethylene glycol-modified distearoyl phosphatidylethanolamine has a molecular weight of 2000-5000 Da.

In some embodiments, the polyethylene glycol-modified distearoyl phosphatidylethanolamine is selected from polyethylene glycol 2000-phosphatidylethanolamine (mPEG2000-DSPE).

In some embodiments, the liposome contains polyethylene glycol-modified distearoyl phosphatidylglycerol.

In some embodiments, the liposome contains polyethylene glycol-modified cholesterol.

In another aspect, in some embodiments, the liposome contains hydrogenated egg phosphatidylcholine, cholesterol, and a distearoyl phosphatidylethanolamine-polyethylene glycol condensate.

In some embodiments, the liposome contains hydrogenated soy phosphatidylcholine, cholesterol, and polyethylene glycol 2000-phosphatidylethanolamine.

In another aspect, in some embodiments, the hydrophilic polymer-modified lipid has a content (relative to the total liposome membrane components, mole fraction) selected from the group consisting of 0.1%-50%, including but not limited to 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50% or any value between any two of the values, preferably 0.5%-20%.

In some embodiments, the liposome contains 10%-80% hydrogenated egg phosphatidylcholine, 1%-60% cholesterol, and 0-50% distearoyl phosphatidylethanolamine-polyethylene glycol condensate.

In another aspect, the phospholipid and steroid in the liposome are in a molar ratio selected from the group consisting of 1:0.01-1:1, including but not limited to 1:0.01, 1:0.02, 1:0.03, 1:0.04, 1:0.05, 1:0.06, 1:0.07, 1:0.08, 1:0.09, 1:0.1, 9:1, 9:2, 9:4, 9:5, 9:7, 9:8, 8:1, 8:3, 8:7, 7:1, 7:2, 7:3, 7:4, 7:5, 7:6, 6:1, 5:1, 5:2, 5:3, 4:1, 3:1, 3:2, 2:1, 4:3, 1:1 or any value between any two of the values, 1:1-3:1, e.g., 3:2.

In some embodiments, the phospholipid and hydrophilic polymer-modified lipid in the liposome are in a molar ratio of 20:1-1:1, including but not limited to 20:1, 19:1, 18:1, 17:1, 16:1, 15:1, 14:1, 13:1, 12:1, 11:1, 10:1, 10:1, 10:1, 10:1, 9:1, 9:2, 9:4, 9:5, 9:7, 9:8, 8:1, 8:3, 8:7, 7:1, 7:2, 7:3, 7:4, 7:5, 7:6, 6:1, 5:1, 5:2, 5:3, 4:1, 3:1, 3:2, 2:1, 4:3, 1:1 or any value between any two of the values, preferably 15:1-2:1, e.g., 9.45:1.

In some embodiments, the active ingredient and the lipid are in a mass ratio (drug-to-lipid ratio) selected from the group consisting of 1:5-1:45, including but not limited to 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39, 1:40, 1:41, 1:42, 1:43, 1:44, 1:45, 1:46, 1:47, 1:48, 1:49, 1:50 or any value between any two of the values, preferably 1:10-1:45, e.g., 1:27.

In another aspect, the liposome of the present disclosure is selected from the group consisting of multilamellar vesicles (MLVs), large unilamellar vesicles (LUVs), small unilamellar vesicles, oligolamellar vesicles (OLVs), phospholipid vesicles (PLVs), and reverse-phase evaporation vesicles (REVs).

In another aspect, in order to improve the encapsulation efficiency of eribulin or a pharmaceutically acceptable salt thereof in the liposome, a solution containing an electrolyte (salt solution) may be added to the outer aqueous phase of the liposome to increase the ionic strength, thereby increasing the encapsulation efficiency. The electrolyte (salt) contained in the outer aqueous phase of the liposome is not particularly limited, but is preferably sodium chloride or potassium chloride, and more preferably sodium chloride. In addition, normal saline may also be used. In addition, the outer aqueous phase of the liposome as a liposome dispersion or the like may contain a sugar, an electrolyte, and/or an amino acid, or may contain a sugar or an electrolyte, and an amino acid. As an example, the sugar is selected from sucrose or trehalose or glucose, the electrolyte is selected from normal saline or sodium chloride or potassium chloride, and the amino acid may be selected from histidine.

In some embodiments, the liposome contains sodium chloride and histidine in the outer aqueous phase.

In some embodiments, the liposome contains sodium chloride and sucrose in the outer aqueous phase.

In some embodiments, the liposome contains sodium chloride, sucrose and histidine in the outer aqueous phase.

In some embodiments, the sugar in the outer aqueous phase of the liposome has a concentration selected from the group consisting of 2%-20%, including but not limited to 2%, 3%, 4%, 5%, 6%, 7%, 8%, 9%, 10%, 11%, 12%, 13%, 14%, 15%, 16%, 17%, 18%, 19%, 20% or any value between any two of the values, preferably 4%-15%.

In some embodiments, the amino acid in the outer aqueous phase of the liposome has a concentration selected from the group consisting of 1-300 mM, which may be 1 mM, 2 mM, 3 mM, 4 mM, 5 mM, 6 mM, 7 mM, 8 mM, 9 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 155 mM, 160 mM, 165 mM, 170 mM, 175 mM, 180 mM, 185 mM, 190 mM, 195 mM, 200 mM, 205 mM, 210 mM, 215 mM, 220 mM, 225 mM, 230 mM, 235 mM, 240 mM, 245 mM, 250 mM, 255 mM, 260 mM, 265 mM, 270 mM, 275 mM, 280 mM, 285 mM, 290 mM, 295 mM, 300 mM or any value between any two of the values, preferably 5-200 mM.

The present disclosure also provides a method for preparing the above liposome, which comprises the following steps: preparing a blank liposome containing one or more selected from the group consisting of ethylenediaminetetraacetic acid, phosphoric acid, pentetic acid and salts thereof in an inner aqueous phase, removing ethylenediaminetetraacetic acid, phosphoric acid, pentetic acid or salts thereof from an outer aqueous phase of the blank liposome, and introducing eribulin or a pharmaceutically acceptable salt thereof into the inner aqueous phase of the blank liposome.

Further, the prepared liposome further contains an ammonium salt in the inner aqueous phase, which may be at least one of ammonium sulfate, ethylammonium sulfate, ammonium ethylenediaminetetraacetate, ammonium chloride, ammonium hydroxide, ammonium acetate, ammonium phosphate, dextran ammonium sulfate and triethyldextran ammonium sulfate, preferably at least one of ammonium sulfate, ammonium chloride and ammonium ethylenediaminetetraacetate, and more preferably ammonium ethylenediaminetetraacetate or ammonium sulfate.

In some embodiments, provided is a method for preparing the above liposome, which comprises the following steps: preparing a blank liposome containing ethylenediaminetetraacetic acid or a salt thereof and an ammonium salt in an inner aqueous phase, removing the ethylenediaminetetraacetic acid or the salt thereof and the ammonium salt from an outer aqueous phase of the blank liposome, and introducing eribulin or a pharmaceutically acceptable salt thereof into the inner aqueous phase of the blank liposome.

In some embodiments, provided is a method for preparing the above liposome, which comprises the following steps: preparing a blank liposome containing phosphoric acid or a salt thereof and an ammonium salt in an inner aqueous phase, removing the phosphoric acid or the salt thereof and the ammonium salt from an outer aqueous phase of the blank liposome, and introducing eribulin or a pharmaceutically acceptable salt thereof into the inner aqueous phase of the blank liposome.

In some embodiments, provided is a method for preparing the above liposome, which comprises the following steps: preparing a blank liposome containing pentetic acid or a salt thereof and an ammonium salt in an inner aqueous phase, removing the pentetic acid or the salt thereof and the ammonium salt from an outer aqueous phase of the blank liposome, and introducing eribulin or a pharmaceutically acceptable salt thereof into the inner aqueous phase of the blank liposome.

In another aspect, in order to achieve the purpose of the present application, a film dispersion method, a reverse-phase evaporation method, a direct hydration method, a surfactant removing method, an ethanol injection method, and a modified ethanol injection method are used to prepare a blank liposome in the present disclosure.

In some embodiments, provided is a method for preparing a blank liposome containing ethylenediaminetetraacetic acid in an inner aqueous phase, which comprises the following steps: 1) preparing an aqueous phase solution of the liposome, 2) preparing the liposome by an ethanol injection method, and 3) replacing or diluting the solution of the liposome in the step 2).

In another aspect, the blank liposome with the required particle size is obtained by processing with a probe sonicator, a high-pressure homogenizer, a microfluidizer, an extruder and other devices.

In some embodiments, the lipid mixture is weighed and dissolved in ethanol, the solution of lipids in ethanol is injected into the above aqueous phase solution of the liposome, the resulting mixture is stirred, ethanol is evaporated off, and the residue is extruded by an extruder to obtain a blank liposome.

The concentration of an ammonium salt in the solution containing the ammonium salt can be adjusted as appropriate according to the amount of eribulin or the like to be encapsulated, and a higher concentration is preferable, preferably 10 mM or more, more preferably 50 mM or more, and even more preferably 100 mM or more. The solution containing the ammonium salt preferably has a pH of 3, 4, 5, 6, 7, 8, 9 or any value between any two of the values, and in view of the balance between the encapsulation efficiency and the stability, more preferably 4-9, and even more preferably 5-8.

In order to adjust the pH of the solution containing the ammonium salt, a pH adjuster may be used. The concentration of each pH adjuster in the solution containing the ammonium salt is not particularly limited, but is preferably 1-300 mM, and more preferably 5-100 mM.

The pH adjuster may be selected from the group consisting of amino acids such as arginine, histidine or glycine; acids such as ethylenediaminetetraacetic acid, hydrochloric acid, phosphoric acid or sulfuric acid; salts such as a sodium salt, a potassium salt, or an ammonium salt of the above acids; basic compounds (alkalis) such as ammonia water, sodium hydroxide or potassium hydroxide; and the like; preferably sodium hydroxide, hydrochloric acid, ammonia water, phosphoric acid, pentetic acid or ethylenediaminetetraacetic acid. In addition, the pH adjuster may be used in combination with 2 or more of the above ammonium salts.

In some embodiments, the pH adjuster is selected from the group consisting of histidine, phosphoric acid, pentetic acid, hydrochloric acid, sulfuric acid, ethylenediaminetetraacetic acid, ammonia water (ammonia), sodium hydroxide, and potassium hydroxide. In some embodiments, the pH adjuster is selected from a base, which is selected from the group consisting of ammonia water (ammonia), sodium hydroxide and potassium hydroxide.

In another aspect, the liposome prepared in the present disclosure is substantially free of citric acid in the inner aqueous phase. The "substantially free of citric acid" means that no citric acid is added. It should be noted that it is sufficient to determine that, for example, the stability of the encapsulation efficiency of the liposome is not due to the addition of citric acid.

In addition, various parameters (e.g., the amount of membrane components, temperature, etc.) in the liposome preparation process can be appropriately adjusted and determined according to the method for preparing the liposome or the composition, particle size and the like of the liposome of interest.

In another aspect, the replacement or dilution operation of the present disclosure may be performed by dialysis, centrifugal separation, gel filtration, and other methods. In addition, eribulin or a pharmaceutically acceptable salt thereof may also be effectively encapsulated in the inner aqueous phase of the liposome by replacing or diluting the outer aqueous phase of the liposome.

The dialysis, centrifugal separation and gel filtration are all conventional operations in the art. For example, dialysis can be performed using a dialysis membrane. The dialysis membrane can be made from polyethersulfone.

If necessary, eribulin or the like may be introduced into the inner aqueous phase of the liposome by a pH gradient. In this case, the inner aqueous phase of the liposome in the liposome dispersion preferably has a pH of 3-9, including 3, 4, 5, 6, 7, 8, 9 or any value between any two of the values, more preferably 4-9, and even more preferably 5-8.

In addition, the outer aqueous phase of the liposome can be adjusted to a pH higher than the pH of the inner aqueous phase of the liposome to give a pH gradient. Preferably, the pH gradient is 1-5, and more preferably 2-3.

Further, by adjusting the pH of the outer aqueous phase of the liposome to be around the pKa of eribulin, the encapsulation efficiency into the liposome can be improved. Preferably, the pH of the outer aqueous phase of the liposome is 7.5-12.5, more preferably 8.5-11.5, and even more preferably 9-10.5 (pKa of eribulin mesylate: 9.6).

In another aspect, the method for preparing the above liposome of the present disclosure further comprises the step of adjusting the pH of the outer aqueous phase of the liposome after drug loading. The pH of the outer aqueous phase adjusted is not particularly limited, but in view of chemical stability of the phospholipid constituting the liposome, is preferably 4-10, including 4, 5, 6, 7, 8, 9, 10 or any value between any two of the values, more preferably 5-9, and even more preferably a neutral pH of 6-8, for example, pH 7.5.

In another aspect, the liposome of the present disclosure is in a solid or liquid form.

In some embodiments, the liposome is liquid, and the liposome composition resulting from the above introduction step can be used directly as a final liposome, or the outer aqueous phase of the liposome of the resulting liposome composition may be adjusted (replaced, etc.) to obtain a final liposome.

In some other embodiments, the liposome is solid, and the liposome composition resulting from the above introduction step can be dried (including but not limited to freeze-dried) to be used as a final solid liposome. The method for drying the liposome composition may include, but is not limited to, freeze-drying, spray drying, and the like.

The present disclosure also provides a pharmaceutical composition comprising the above liposome or prepared from the above liposome. Further, the pharmaceutical composition further comprises a buffer, which is preferably selected from at least one of an acetate buffer, a histidine buffer, a phosphate buffer, a succinate buffer and a 4-hydroxyethylpiperazine ethanesulfonate buffer, more preferably at least one of a histidine buffer, a phosphate buffer (such as PBS) and a 4-hydroxyethylpiperazine ethanesulfonate buffer (such as HEPES), e.g., a histidine buffer.

"Buffer" refers to a buffer that resists changes in pH by the action of its acid-base conjugate components. Examples of a buffer that controls the pH in an appropriate range include acetate, succinate, gluconate, histidine, oxalate, lactate, phosphate, citrate, tartrate, fumarate, glycylglycine and other organic acid buffers.

"Histidine buffer" is a buffer containing histidine ions. Examples of the histidine buffer include histidine-hydrochloride, histidine-acetate, histidine-phosphate, histidine-sulfate, and the like. The preferred histidine buffer is a histidine-hydrochloride buffer. The histidine-hydrochloride buffer is formulated with histidine and hydrochloric acid, or with histidine and histidine hydrochloride.

"Succinate buffer" is a buffer containing succinate ions. Examples of the succinate buffer include succinic acid-sodium succinate, succinic acid-histidine salt, succinic acid-potassium succinate, succinic acid-calcium succinate, and the like. The preferred succinate buffer is a succinic acid-sodium succinate buffer.

"Phosphate buffer" is a buffer containing phosphate ions. Examples of the phosphate buffer include disodium hydrogen phosphate-sodium dihydrogen phosphate, disodium hydrogen phosphate-potassium dihydrogen phosphate, and the like. The preferred phosphate buffer is disodium hydrogen phosphate-sodium dihydrogen phosphate buffer.

"Acetate buffer" is a buffer containing acetate ions. Examples of the acetate buffer include acetic acid-sodium acetate, acetic acid-histidine salt, acetic acid-potassium acetate, acetic acid-calcium acetate, acetic acid-magnesium acetate, and the like. The preferred acetate buffer is an acetic acid-sodium acetate buffer.

In another aspect, the pharmaceutical composition of the present disclosure further comprises a sugar or an electrolyte.

As an example, the sugar is selected from sucrose or trehalose or glucose, the electrolyte is selected from normal saline or sodium chloride or potassium chloride, and the amino acid may be selected from histidine.

In some embodiments, the pharmaceutical composition further comprises sodium chloride and histidine.

In some embodiments, the pharmaceutical composition further comprises sodium chloride and sucrose.

In some embodiments, the pharmaceutical composition further comprises sodium chloride, sucrose, and histidine.

In some embodiments, the pharmaceutical composition comprises normal saline and sucrose. In some embodiments, the pharmaceutical composition comprises normal saline, sucrose and histidine.

In some embodiments, the sugar has a concentration selected from the group consisting of 2%-20%, including but not limited to 2%, 3%, 4%, 5%, 6%, 7%, 8%, 9%, 10%, 11%, 12%, 13%, 14%, 15%, 16%, 17%, 18%, 19%, 20% or any value between any two of the values, preferably 4%-15%.

In some embodiments, the amino acid has a concentration selected from the group consisting of 1-300 mM, which may be 1 mM, 2 mM, 3 mM, 4 mM, 5 mM, 6 mM, 7 mM, 8 mM, 9 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 155 mM, 160 mM, 165 mM, 170 mM, 175 mM, 180 mM, 185 mM, 190 mM, 195 mM, 200 mM, 205 mM, 210 mM, 215 mM, 220 mM, 225 mM, 230 mM, 235 mM, 240 mM, 245 mM, 250 mM, 255 mM, 260 mM, 265 mM, 270 mM, 275 mM, 280 mM, 285 mM, 290 mM, 295 mM, 300 mM or any value between any two of the values, preferably 5-200 mM.

In addition, the pH of the pharmaceutical composition affects the stability of the drug. In some embodiments, the pharmaceutical composition has a pH selected from the group consisting of 5.0-8.0, including but not limited to 5.0, 5.5, 6.0, 6.5, 7.0, 7.5, 8.0 or any value between any two of the values, preferably 5.5-7.5.

In another aspect, the pharmaceutical composition of the present disclosure further comprises an antioxidant, which is selected from the group consisting of, but not limited to, $\alpha$-tocopherol, $\alpha$-tocopheryl succinate, $\alpha$-tocopheryl acetate, ascorbic acid, sodium bisulfite, sodium sulfite, sodium metabisulfite, and L-cysteine. In some embodiments, the antioxidant is added in an amount of 0-0.5%, including but not limited to 0%, 0.1%, 0.2%, 0.3%, 0.4% and 0.5%.

In some embodiments, the pharmaceutical composition is a liquid formulation, and the liposome composition resulting from the above introduction step can be directly used as a final liposome composition (pharmaceutical composition), or the outer aqueous phase of the liposome of the resulting liposome composition may be adjusted (replaced, etc.) to obtain a final liposome composition (pharmaceutical composition).

In some other embodiments, the pharmaceutical composition is a solid formulation, and the liposome composition resulting from the above introduction step can be dried to be used as a final solid liposome composition (pharmaceutical composition). The method for drying the liposome composition may include, but is not limited to, freeze-drying, spray drying, and the like.

In another aspect, the pharmaceutical composition is a solid formulation, and the pharmaceutical composition can be dissolved and suspended in an appropriate solvent to be used as a liquid formulation. The solvent may be appropriately set according to the purpose of use of the liposome composition. For example, when the pharmaceutical composition is used as an injection, the solvent is preferably sterilized distilled water.

The present disclosure also provides a lyophilized composition, which is obtained by freeze-drying the above pharmaceutical composition, or reconstituted in a liquid medium to obtain the above pharmaceutical composition, wherein the liquid medium used for the reconstitution is selected from the group consisting of normal saline, water for injection, a glucose injection, and a glucose and sodium chloride injection.

In another aspect, the liposome/pharmaceutical composition/lyophilized composition of the present disclosure is quite stable during storage, for example, as determined by the percentage of entrapped/encapsulated active ingredient released outside the liposome of the present disclosure after a period of time from initial loading with the liposome to active ingredient still remaining in the liposome. In some embodiments, the liposome/pharmaceutical composition of the present disclosure is stable for at least 3 months at 25 °C. For example, at least 70% of the active ingredient remains encapsulated in the liposome after 3 months at 25 °C after initial loading with the active ingredient. For example, at least 75% of the active ingredient remains encapsulated in the liposome after 3 months at 25 °C after initial loading with the active ingredient. For example, at least 80% of the active ingredient remains encapsulated in the liposome after 3 months at 25 °C after initial loading with the active ingredient. For example, at least 85% of the active ingredient remains encapsulated in the liposome after 3 months at 25 °C after initial loading with the active ingredient. For example, at least 90% of the active ingredient remains encapsulated in the liposome after 3 months at 25 °C after initial loading with the active ingredient. For example, at least 95% or more of the active ingredient remains encapsulated in the liposome after 3 months at 25 °C after initial loading with the active ingredient.

In another aspect, for example, less than 10% of the entrapped/encapsulated active ingredient is released after 3 months at 25 °C after initial loading with the active ingredient. For example, less than 15% of the entrapped/encapsulated active ingredient is released after 3 months at 25 °C after initial loading with the active ingredient. For example, less than 20% of the entrapped/encapsulated active ingredient is released after 3 months at 25 °C after initial loading with the active ingredient. For example, less than 30% of the entrapped/encapsulated active ingredient is released after 3 months at 25 °C after initial loading with the active ingredient.

In some embodiments, the liposome/pharmaceutical composition/lyophilized composition of the present disclosure is stable for at least 3 months at 2-8 °C. For example, less than 10% of the entrapped entity is released 6 months after initial loading with the entity. In some embodiments, the liposome/pharmaceutical composition of the present disclosure is stable for at least 2 years at 4 °C. For example, less than 20% of the entrapped entity is released 2 years after initial loading with the entity.

For example, in the case of administration of a liposomal antitumor drug to a mammal, such as a human, it is advantageous that the liposome-entrapped entity remains encapsulated in the liposome until the liposome reaches the tumor. The liposome/pharmaceutical composition/lyophilized composition of the present invention shows surprising stability against release (leakage) of the entrapped active ingredient under *in vivo* conditions, e.g., in the blood of a mammal such as a human.

In some embodiments, the active ingredient in the above liposome/pharmaceutical composition/lyophilized composition provides a sustained release of the drug in a mammal for not less than 12 h, preferably for not less than 24 h, more preferably for not less than 48 h, and most preferably for not less than 72 h.

The liposome of the present disclosure improves the therapeutic efficacy of the entrapped/encapsulated active ingredient as well as reduces the toxicity of the active ingredient. In general, the activity of the active ingredient entrapped/encapsulated in the liposome of the present invention in a mammal, e.g. the antitumor activity of eribulin mesylate, is at least equal or 2-20 fold (including 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, and 20-fold) higher than that of the active ingredient in the same amount by its conventional non-liposomal formulation, e.g., eribulin injection (Halaven), while the toxicity of the active ingredient entrapped/encapsulated in the liposome is at least 2-fold, at least 4-fold or at least 4-fold lower than that of the active ingredient in the same amount by its conventional non-liposomal formulation, e.g., eribulin injection (Halaven).

The liposome/pharmaceutical composition of the present disclosure can be administered in a variety of ways including, but not limited to, topical route, parenteral route, and other routes. The composition may include ophthalmic and injectable dosage forms, and may include medical diagnostic products.

In another aspect, the present disclosure also provides use of the liposome/pharmaceutical composition/lyophilized composition described above or a liposome/pharmaceutical composition/lyophilized composition prepared by the method described above in preparing a medicament for treating or preventing tumors, wherein the cancer is preferably breast cancer, ovarian cancer, cervical cancer, uterine cancer, prostate cancer, renal cancer, urethral cancer, bladder cancer, liver cancer, stomach cancer, endometrial cancer, salivary gland cancer, pancreatic cancer and lymphoma.

Terminology:

Drug-to-lipid ratio refers to the weight ratio of the drug to the phospholipid.

Lipid content, such as steroid content, refers to the percentage of the total number of moles of the phospholipid component in the liposomal bilayer.

Liposome refers to microscopic lipid vesicles composed of a bilayer phospholipid or any similar amphiphilic lipid encapsulating an inner aqueous medium. The liposome of the present disclosure may be multilamellar vesicles (MLVs), large unilamellar vesicles (LUVs), or small unilamellar vesicles, with the size usually ranging from 30 mM to 200 mM. In addition, there is no particular limitation on the liposome membrane structure in the present disclosure. Liposome membrane refers to a bilayer phospholipid that separates the inner aqueous medium from the outer aqueous medium.

Inner aqueous phase of liposome refers to an aqueous region surrounded by a lipid bilayer of a liposome, and is used in the same sense as the "inner aqueous phase" and the "liposomal inner aqueous phase". When the liposome is dispersed in a liquid, "outer aqueous phase of the liposome" refers to a region not surrounded by a lipid bilayer of a liposome (i.e., a region excluding the inner aqueous phase and the lipid bilayer).

Liposome composition refers to a composition containing a liposome and eribulin mesylate in the inner aqueous phase of the liposome. In the present disclosure, the liposome composition includes compositions in solid and liquid forms.

Blank liposome refers to a liposome free of the active ingredient to be loaded, such as eribulin of the present disclosure, in the inner aqueous phase.

Encapsulation efficiency refers to the percentage of an encapsulated substance (such as a drug) in a liposome suspension relative to the total amount of the drug. It is an important index for controlling the quality of liposomes and nanoparticles, and reflects the degree of encapsulation of the drug by a carrier.

The encapsulation efficiency includes the determination of the encapsulation percentage and the encapsulation volume, and in general, the encapsulation percentage (EN%) is mainly investigated in the literatures. The expression is EN% = (1 - Cf/Ct) × 100%. In the expression, Cf is the amount of free drug; Ct is the total amount of drug in the liposome suspension.

$$\text{Encapsulation efficiency}(EN\%)=1-\frac{\text{Amount of free drug}}{\text{Total amount of drug in liposome suspension}}\times 100\%$$

The encapsulation efficiency can be determined by conventional determination methods for encapsulation efficiency, such as HPLC. In some embodiments, the HPLC conditions are as follows: chromatographic column: ODS-A, 150 × 4.6 mm, 5 $\mu$m; mobile phase: methanol-acetic acid-triethylamine; flow rate: 1.0 mL/min; column temperature: 25 °C; injection volume: 20 $\mu$L.

In the present disclosure, "polymer molecular weight" is taken as number-average molecular weight and refers to the statistical average of the molecular weights of all polymeric chains in a sample, which is defined as follows, where $M_i$ represents the molecular weight of a single chain and $N_i$ represents the number of chains with the corresponding molecular weight. $M_n$ can be predicted by the polymerization mechanism and determined by a method for measuring the number of molecules in a sample of a given mass, e.g., a colligative method such as terminal analysis. If $M_n$ is used to characterize the molecular weight distribution, there are an equal number of molecules distributed on both sides of $M_n$.

In the present disclosure, the value such as the encapsulation efficiency is the data obtained by determination and calculation, and there is inevitably a certain degree of error. In general, $\pm$ 10% falls within a reasonable error range. There is a certain degree of error change according to the context of the place where it is used, and the error change is no more than $\pm$ 10%, and may be $\pm$ 9%, $\pm$ 8%, $\pm$ 7%, $\pm$ 6%, $\pm$ 5%, $\pm$ 4%, $\pm$ 3%, $\pm$ 2% or $\pm$ 1%, preferably $\pm$ 5%.

## DETAILED DESCRIPTION

Example 1

| Formula | Amount |
|---|---|
| HSPC | 1215mg |
| CHOL | 398.09mg |
| mPEG2000-DSPE | 460.29mg |
| Eribulin mesylate | 45mg |

The formula amount of lipid mixture was taken, added to absolute ethanol, and dissolved at 60 °C. The solution of lipids in ethanol was injected into the inner aqueous phase solution of the liposome (300 mM ammonium sulfate/60 mM citric acid), and the resulting mixture was stirred for 15-30 min. Ethanol was removed under reduced pressure, and the residue was extruded and sized by an extruder to obtain a blank liposome.

The blank liposome was dialyzed (tangential flow filtration) against 180 mL of 0.9% sodium chloride/10 mM histidine buffer (pH = 7.5), and when most of the ammonium sulfate was removed from the outer aqueous phase, the pH of the outer aqueous phase of the liposomes was adjusted to 10.5 with sodium hydroxide.

The formula amount of eribulin mesylate was dissolved with an aqueous solution of 0.9% sodium chloride/10 mM histidine (pH 7.5), and the solution was stirred to obtain a 5 mg/mL eribulin mesylate solution.

The blank liposome (with outer aqueous phase at pH 10.5) was mixed with the eribulin mesylate solution, and the mixture was incubated at 60 °C to allow eribulin mesylate to be introduced into the liposome in the liposome. The outer aqueous phase of liposome was adjusted to pH 7.5, and the mixture was concentrated, and brought to a certain volume.

Determination of encapsulation efficiency:

The liposome encapsulating the active ingredient was centrifuged at 13,000 rpm for 10-30 min, and the concentration of the active ingredient in the supernatant was determined by HPLC, and the corresponding value was calculated according to the encapsulation efficiency expression.

The encapsulation efficiency of eribulin mesylate was 95.70%.

Example 2

| Formula | Amount |
|---|---|
| HSPC | 1215mg |
| CHOL | 398.09mg |
| mPEG2000-DSPE | 460.29mg |
| Eribulin mesylate | 45mg |

The formula amount of lipid mixture was taken, added to absolute ethanol, and dissolved at 60 °C. The solution of lipids in ethanol was injected into the inner aqueous phase solution of the liposome (300 mM ammonium sulfate/60 mM ethylenediaminetetraacetic acid), and the resulting mixture was stirred for 15-30 min. Ethanol was removed under reduced pressure, and the residue was extruded and sized by an extruder to obtain a blank liposome.

The blank liposome was dialyzed (tangential flow filtration) against 180 mL of 0.9% sodium chloride/10 mM histidine buffer (pH = 7.5), and when most of the ammonium sulfate was removed from the outer aqueous phase, and the pH of the outer aqueous phase of the liposomes was adjusted to 10.5 with sodium hydroxide.

The formula amount of eribulin mesylate was dissolved with an aqueous solution of 0.9% sodium chloride/10 mM histidine (pH 7.5), and the solution was stirred to obtain a 5 mg/mL eribulin mesylate solution.

The blank liposome (with outer aqueous phase at pH 10.5) was mixed with the eribulin mesylate solution, and the mixture was incubated at 60 °C to introduce eribulin mesylate into the inner aqueous phase of the liposome. The outer aqueous phase of liposome was adjusted to pH 7.5, concentrated, and brought to a certain volume.

Determination of encapsulation efficiency:

The encapsulation efficiency of eribulin mesylate was 96.85%.

Example 3

| Formula | Amount |
|---|---|
| HSPC | 1215mg |
| CHOL | 398.09mg |
| mPEG2000-DSPE | 460.29mg |
| Eribulin mesylate | 45mg |

The formula amount of lipid mixture was taken, added to absolute ethanol, and dissolved at 60 °C. The solution of lipids in ethanol was injected into the inner aqueous phase solution of the liposome (300 mM ammonium sulfate/60 mM phosphoric acid), and the resulting mixture was stirred for 15-30 min. Ethanol was removed under reduced pressure, and the residue was extruded and sized by an extruder to obtain a blank liposome.

The blank liposome was dialyzed (tangential flow filtration) against 180 mL of 0.9% sodium chloride/10 mM histidine buffer (pH = 7.5), and when most of the ammonium sulfate was removed from the outer aqueous phase, and the pH of the outer aqueous phase of the liposomes was adjusted to 10.5 with sodium hydroxide.

The formula amount of eribulin mesylate was dissolved with an aqueous solution of 0.9% sodium chloride/10 mM histidine (pH 7.5), and the solution was stirred to obtain a 5 mg/mL eribulin mesylate solution.

The blank liposome (with outer aqueous phase at pH 10.5) was mixed with the eribulin mesylate solution, and the mixture was incubated at 60 °C to introduce eribulin mesylate into the inner aqueous phase of the liposome. The outer aqueous phase of liposome was adjusted to pH 7.5, concentrated, and brought to a certain volume.

Determination of encapsulation efficiency:

The encapsulation efficiency of eribulin mesylate was 99.46%.

Example 4

| Formula | Amount |
|---|---|
| HSPC | 1215mg |
| CHOL | 398.09mg |
| mPEG2000-DSPE | 460.29mg |
| Eribulin mesylate | 45mg |

The formula amount of lipid mixture was taken, added to absolute ethanol, and dissolved at 60 °C. The solution of lipids in ethanol was injected into the inner aqueous phase solution of the liposome (300 mM ammonium sulfate/60 mM tartaric acid), and the resulting mixture was stirred for 15-30 min. Ethanol was removed under reduced pressure, and the residue was extruded and sized by an extruder to obtain a blank liposome.

The blank liposome was dialyzed (tangential flow filtration) against 180 mL of 0.9% sodium chloride/10 mM histidine buffer (pH = 7.5), and when most of the ammonium sulfate was removed from the outer aqueous phase, and the pH of the outer aqueous phase of the liposomes was adjusted to 10.5 with sodium hydroxide.

The formula amount of eribulin mesylate was dissolved with an aqueous solution of 0.9% sodium chloride/10 mM histidine (pH 7.5), and the solution was stirred to obtain a 5 mg/mL eribulin mesylate solution.

The blank liposome (with outer aqueous phase at pH 10.5) was mixed with the eribulin mesylate solution, and the mixture was incubated at 60 °C to introduce eribulin mesylate into the inner aqueous phase of the liposome. The outer aqueous phase of liposome was adjusted to pH 7.5, concentrated, and brought to a certain volume.

Determination of encapsulation efficiency:

The encapsulation efficiency of eribulin mesylate was 94.92%.

Example 5

| Formula | Amount |
|---|---|
| HSPC | 1215mg |
| CHOL | 398.09mg |
| mPEG2000-DSPE | 460.29mg |
| Eribulin mesylate | 45mg |

The formula amount of lipid mixture was taken, added to absolute ethanol, and dissolved at 60 °C. The solution of lipids in ethanol was injected into the inner aqueous phase solution of the liposome (300 mM ammonium sulfate/60 mM aconitic acid), and the resulting mixture was stirred for 15-30 min. Ethanol was removed under reduced pressure, and the residue was extruded and sized by an extruder to obtain a blank liposome.

The blank liposome was dialyzed (tangential flow filtration) against 180 mL of 0.9% sodium chloride/10 mM histidine buffer (pH = 7.5), and when most of the ammonium sulfate was removed from the outer aqueous phase, and the pH of the outer aqueous phase of the liposomes was adjusted to 10.5 with sodium hydroxide.

The formula amount of eribulin mesylate was dissolved with an aqueous solution of 0.9% sodium chloride/10 mM histidine (pH 7.5), and the solution was stirred to obtain a 5 mg/mL eribulin mesylate solution.

The blank liposome (with outer aqueous phase at pH 10.5) was mixed with the eribulin mesylate solution, and the mixture was incubated at 60 °C to introduce eribulin mesylate into the inner aqueous phase of the liposome. The outer aqueous phase of liposome was adjusted to pH 7.5, concentrated, and brought to a certain volume.

Determination of encapsulation efficiency:

The encapsulation efficiency of eribulin mesylate was 98.02%.

Test Example 1

The samples obtained in Examples 1-5 described above were separately placed at 2-8 °C or 25 °C for investigating the stability of the encapsulation efficiency. The related data are shown in the following table:

Table 1

| Examples | | 1 | 2 | 3 | 4 | 5 |
|---|---|---|---|---|---|---|
| Starting point (%) | | 95.70 | 96.85 | 99.46 | 94.92 | 98.02 |
| 2-8 °C | 1 month (%) | 95.31 | 96.84 | N/A | N/A | 94.25 |
| | 2 months (%) | 98.04 | 97.29 | 98.80 | 95.35 | 89.93 |
| | 3 months (%) | 98.10 | 96.64 | 99.18 | 94.24 | 89.37 |

(continued)

| Examples | | 1 | 2 | 3 | 4 | 5 |
|---|---|---|---|---|---|---|
| 25°C | 15 days (%) | 93.49 | 94.71 | 99.25 | 96.61 | N/A |
| | 1 month (%) | 81.43 | 93.31 | 93.64 | 92.01 | 73.40 |
| | 2 months (%) | 83.08 | 93.25 | 90.93 | 83.20 | 62.65 |
| | 3 months (%) | 44.06 | 91.64 | 91.45 | 74.67 | 56.29 |

Test Example 2

The samples obtained in Examples 1-5 described above were separately placed at 2-8 °C or 25 °C for investigating the content. The related data are shown in Table 2 below:

Table 2

| Examples | | 1 | 2 | 3 | 4 | 5 |
|---|---|---|---|---|---|---|
| Starting point (%) | | 0.5368 | 0.5347 | 0.5715 | 0.5225 | 0.5136 |
| 2-8 °C | 1 month (%) | 0.5602 | 0.5454 | N/A | N/A | 0.4804 |
| | 2 months (%) | 0.5401 | 0.5319 | 0.5799 | 0.5218 | 0.4922 |
| | 3 months (%) | 0.5586 | 0.5582 | 0.5606 | 0.5551 | 0.4927 |
| 25°C | 15 days (%) | 0.5697 | 0.5593 | 0.5734 | 0.5256 | N/A |
| | 1 month (%) | 0.5437 | 0.5595 | 0.5512 | 0.5375 | 0.4961 |
| | 2 months (%) | 0.5577 | 0.5445 | 0.5544 | 0.5378 | 0.5005 |
| | 3 months (%) | 0.5859 | 0.5583 | 0.5333 | 0.5455 | 0.5117 |

Conclusion: as can be seen from Table 2, all of the liposomes with different compositions have good content stability. It is more important to determine whether the drug will be released from the liposome during the *in vitro* storage period, and it can be seen from Table 1 that the stability of the encapsulation efficiency of the liposome containing phosphoric acid or ethylenediammoniumtetraacetic acid in the inner aqueous phase is superior as compared to that of the liposome containing citric acid or tartaric acid or aconitic acid in the inner aqueous phase, especially under accelerated conditions (25 °C). Therefore, it is expected that the composition containing such liposomes is suitable for long-term storage under mild conditions.

Example 6

The pharmaceutical compositions (liposomes) A, B, C and D as shown in Table 3 below were prepared according to the method of Example 1. Female Balb/c nude mice (Beijing Vital River Laboratory Animal Technology Co., Ltd.) were subcutaneously inoculated with tumor cells (human pharyngeal squamous carcinoma cells FaDu) on the right dorsal side.

Reagent: pharmaceutical compositions (liposomes) A, B, C and D, eribulin mesylate injection (produced in-house)
Mode of administration: once a week, 0.15 mg/mL $\times$ 10 mL, intravenous injection.

After a certain period of time (0.5 h, 2 h, 6 h, 24 h and 48 h) after the administration, blood was collected or tumor tissues were removed. The blood samples were centrifuged within 1 h after the collection (centrifugation condition: about 11,000 rpm at 2-8 °C for 5 min), and could be initially placed on ice after the collection and placed in a refrigerator at -80 °C for long-term storage. After being removed, the tumor tissues were washed with PBS and wiped with absorbent paper, and the weight of the tissues was measured and recorded. The tissues were loaded into a test tube, cooled in ice water and stored at -80 °C for later analysis.

The amount of eribulin mesylate in plasma and tumor tissues was determined by LC/MS.

The results of plasma PK data and tumor tissue PK data for eribulin mesylate are shown in Tables 4 and 5, respectively.

Table 3

| Formula | A | B | C |
|---|---|---|---|
| HSPC | 13.5mg/mL | 13.5mg/mL | 13.5mg/mL |
| CHOL | 4.42 mg/mL | 4.42 mg/mL | 4.42 mg/mL |
| mPEG2000-DSPE | 5.11 mg/mL | 5.11 mg/mL | 5.11 mg/mL |
| Drug/lipid ratio | 1:27 | 1:27 | 1:27 |
| Inner aqueous phase | Ammonium sulfate/ ethylenediaminetetraacetic acid | Ammonium sulfate/ phosphoric acid | Ammonium sulfate/ citric acid |
| Outer aqueous phase | Sodium chloride/histidine | Sodium chloride/histidine | Sodium chloride/ histidine |

Table 4 Plasma PK data

| Group | $T_{max}$ (h) | $C_{max}$ (ug/mL) | $AUC_{0-t}$ (h*ug/mL) | $T_{1/2}$ (h) | Ratio |
|---|---|---|---|---|---|
| Eribulin mesylate injection | 0.5 | 0.154 | 0.654 | 3.38 | -- |
| A | 0.5 | 20.3 | 304 | 8.17 | 465 |
| B | 0.5 | 21.1 | 285 | 8.71 | 436 |
| C | 0.5 | 21.1 | 307 | 8.94 | 469 |
| Ratio: $AUC_{liposome}/AUC_{eribulin}$ mesylate injection | | | | | |

Table 5. Tumor tissue PK data

| Group | $T_{max}$ (h) | $C_{max}$ (ug/mL) | $AUC_{0-t}$ (h*ug/mL) | $T_{1/2}$ (h) | Ratio |
|---|---|---|---|---|---|
| Eribulin mesylate injection | 2 | 0.272 | 4.05 | 16.6 | -- |
| A | 24 | 1.31 | 43 | NA | 10.6 |
| B | 6 | 1.2 | 40.6 | 39.6 | 10.0 |
| C | 24 | 0.936 | 36.7 | NA | 9.1 |
| Ratio: $AUC_{liposome}/AUC_{eribulin\ mesylate\ injection}$ | | | | | |

Conclusion: compared with eribulin mesylate, the pharmaceutical compositions A, B and C show increased AUC and significantly improved release half-cycle in plasma and tumor tissues. In tumor tissues, compared with the liposome with citric acid as the inner aqueous phase, the pharmaceutical compositions A and B have superior $C_{max}$ and higher $AUC_{liposome}/AUC_{eribulin\ mesylate}$ injection.

**Claims**

1. A liposome, containing eribulin or a pharmaceutically acceptable salt thereof as an active compound, and one or more selected from the group consisting of ethylenediaminetetraacetic acid, phosphoric acid, pentetic acid and salts thereof, in an inner aqueous phase.

2. The liposome according to claim 1, further containing an ammonium salt, preferably ammonium sulfate, ethylammonium sulfate, ammonium ethylenediaminetetraacetate, ammonium chloride, ammonium hydroxide, ammonium acetate, ammonium phosphate, triethylammonium sucrose octasulfate, dextran ammonium sulfate or triethyldextran ammonium sulfate, more preferably ammonium sulfate, ammonium phosphate, ammonium chloride or ammonium ethylenediaminetetraacetate, and most preferably ammonium sulfate, in the inner aqueous phase.

3. The liposome according to claim 1 or 2, wherein the active compound is selected from eribulin mesylate.

4. The liposome according to any one of claims 1-3, wherein the ethylenediaminetetraacetic acid, phosphoric acid, pentetic acid or salts thereof has a concentration selected from the group consisting of 1-300 mM, preferably 20-200 mM.

5. The liposome according to claim 3, wherein the ammonium salt has a concentration selected from the group consisting of 10-400 mM, preferably 20-300 mM.

6. The liposome according to any one of claims 1-5, wherein the active compound has a concentration selected from the group consisting of 0.01-100 mg/mL, preferably 0.05-5 mg/mL.

7. The liposome according to any one of claims 1-6, wherein the liposome is substantially free of citric acid in the inner aqueous phase.

8. The liposome according to any one of claims 1-7, further containing a phospholipid selected from at least one of dipalmitoyl phosphatidylcholine (DPPC), distearoyl phosphatidylcholine (DSPC), dimyristoyl phosphatidylcholine (DMPC), 1-palmitoyl-2-linoleoyl-sn-glycero-3-phosphatidylcholine (PLPC), 1,2-dioleoyl-sn-glycero-3-phosphocholine (DOPC), egg yolk phosphatidylcholine (EPC), dilauroyl phosphatidylcholine (DLPC), hydrogenated egg phosphatidylcholine (HEPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoylphosphatidylcholine (SPPC), palmitoyl oleoylphosphatidylcholine (POPC), lysophosphatidylcholine, dilinoleoyl phosphatidylcholine, distearoyl phosphatidylethanolamine (DSPE), dimyristoyl phosphatidylethanolamine (DMPE), dipalmitoyl phosphatidylethanolamine (DPPE), dioleoyl phosphatidylglycerol (DOPG), dimyristoyl phosphatidylglycerol (DMPG), distearoyl phosphatidylglycerol (DSPG), dipalmitoyl glycerophosphoglycerol (DPPG), dipalmitoyl phosphatidylserine (DPPS), 1,2-dioleoyl-sn-glycero-3-phosphatidylserine (DOPS), dimyristoyl phosphatidylserine (DMPS), distearoyl phosphatidylserine (DSPS), 1,2-dipalmitoyl-sn-glycero-3-phosphatidic acid (DPPA), 1,2-dioleoyl-sn-glycero-3-phosphatidic acid (DOPA), 1,2-dimyristoyl-sn-glycero-3-phosphatidic acid (DMPA), 1,2-distearoyl-sn-glycero-3-phosphatidic acid (DSPA), dipalmitoyl phosphatidylinositol (DPPI), 1,2-dioleoyl-sn-glycero-3-phosphatidylinositol (DOPI), dimyristoyl phosphatidylinositol (DMPI), and distearoyl phosphatidylinositol (DSPI), preferably one or more of hydrogenated egg phosphatidylcholine, distearoyl phosphatidylcholine, dipalmitoyl phosphatidylcholine, distearoyl phosphatidylcholine, 1,2-dioleoyl-sn-glycerol-3-phosphocholine and dimyristoyl phosphatidylcholine, wherein the hydrogenated egg phosphatidylcholine is preferably hydrogenated soy phosphatidylcholine.

9. The liposome according to claim 8, wherein the phospholipid has a content selected from the group consisting of 10%-80%, preferably 30%-70%.

10. The liposome according to any one of claims 1-9, further containing a steroid, preferably cholesterol.

11. The liposome according to claim 10, wherein the steroid has a content selected from the group consisting of 1%-60%, preferably 10%-40%.

12. The liposome according to any one of claims 1-11, further containing a hydrophilic polymer-modified lipid, preferably polyethylene glycol-modified distearoyl phosphatidylethanolamine, polyethylene glycol-modified distearoyl phosphatidylglycerol or polyethylene glycol-modified cholesterol, and more preferably polyethylene glycol 2000-phosphatidylethanolamine.

13. The liposome according to claim 12, wherein the hydrophilic polymer-modified lipid has a content selected from the group consisting of 0.1%-50%, preferably 0.5%-20%, e.g., 6%.

14. The liposome according to any one of claims 1-13, containing a phospholipid, a steroid and a hydrophilic polymer-modified lipid, preferably hydrogenated egg phosphatidylcholine, cholesterol and polyethylene glycol-modified distearoyl phosphatidylethanolamine, preferably hydrogenated soy phosphatidylcholine, cholesterol and polyethylene glycol 2000-phosphatidylethanolamine.

15. The liposome according to any one of claims 10-14, wherein the phospholipid and the steroid are in a molar ratio selected from the group consisting of 1:0.01-1:1, preferably 1:1-3:1, e.g., 3:2.

16. The liposome according to any one of claims 13-15, wherein the phospholipid and the hydrophilic polymer-modified lipid are in a molar ratio selected from the group consisting of 20:1-1:1, preferably 15:1-2:1, e.g., 9.45:1.

17. The liposome according to any one of claims 1-16, wherein the active ingredient and the lipid are in a mass ratio selected from the group consisting of 1:5-1:50, preferably 1:10-1:45, e.g., 1:27.

18. The liposome according to any one of claims 1-17, containing a phospholipid, cholesterol and a distearoyl phosphatidylethanolamine-polyethylene glycol condensate, and containing eribulin or a pharmaceutically acceptable salt thereof as an active compound, ethylenediaminetetraacetic acid, phosphoric acid, pentetic acid or salts thereof, and an ammonium salt, in the inner aqueous phase.

19. A method for preparing the liposome according to any one of claims 1-18, comprising: preparing a blank liposome containing one or more selected from the group consisting of ethylenediaminetetraacetic acid, phosphoric acid, pentetic acid and salts thereof in an inner aqueous phase, removing the ethylenediaminetetraacetic acid, phosphoric acid, pentetic acid or salts thereof from an outer aqueous phase of the blank liposome, and introducing eribulin or a pharmaceutically acceptable salt thereof into the inner aqueous phase of the blank liposome.

20. A pharmaceutical composition comprising the liposome according to any one of claims 1-18 or prepared from the liposome according to any one of claims 1-18, wherein further, the pharmaceutical composition preferably further comprises a buffer, preferably selected from at least one of an acetate buffer, a histidine buffer, a phosphate buffer, a succinate buffer and a 4-hydroxyethylpiperazine ethanesulfonate buffer, more preferably at least one of a histidine buffer, a phosphate buffer and a 4-hydroxyethylpiperazine ethanesulfonate buffer, e.g., a histidine buffer.

21. The pharmaceutical composition according to claim 20, further comprising a sugar or an electrolyte, preferably at least one of sodium chloride, trehalose and sucrose.

22. The pharmaceutical composition according to claim 21, wherein the sugar has a concentration selected from the group consisting of 2%-20%, preferably 4%-15%.

23. The pharmaceutical composition according to any one of claims 20-22, wherein the buffer has a concentration selected from the group consisting of 1-300 mM, preferably 5-200 mM.

24. The pharmaceutical composition according to any one of claims 20-22, having a pH of 5.0-8.0, preferably 5.5-7.5.

25. A lyophilized composition obtained by freeze-drying the pharmaceutical composition according to any one of claims 20-24, or reconstituted in a liquid medium to obtain the pharmaceutical composition according to any one of claims 20-24, wherein the liquid medium used for the reconstitution is selected from the group consisting of normal saline, water for injection, a glucose injection, and a glucose and sodium chloride injection.

26. The liposome according to any one of claims 1-18 or the pharmaceutical composition according to any one of claims 20-24 or the lyophilized composition according to claim 25, wherein at least 70% or at least 80% of the active ingredient remains encapsulated in the liposome, preferably at least 85% of the active ingredient remains encapsulated in the liposome after 3 months at 25 °C.

27. The liposome according to any one of claims 1-18 or the pharmaceutical composition according to any one of claims 20-24 or the lyophilized composition according to claim 25, wherein the composition has a toxicity for a mammal being at least two-fold, preferably at least four-fold lower than the composition in a free non-liposomal form.

28. The liposome according to any one of claims 1-18 or the pharmaceutical composition according to any one of claims 20-24 or the lyophilized composition according to claim 25, wherein the active ingredient has a half-release period in the blood of a mammal of at least 5 h, preferably at least 7 h, e.g., 8 h.

29. The liposome according to any one of claims 1-18 or the pharmaceutical composition according to any one of claims 20-24 or the lyophilized composition according to claim 25, wherein the active ingredient provides a sustained release of the drug in a mammal for not less than 12 h, preferably for not less than 24 h, more preferably for not less than 48 h, and most preferably for not less than 72 h.

30. Use of the liposome according to any one of claims 1-18 or the pharmaceutical composition according to any one of claims 20-24 or the lyophilized composition according to claim 25 in preparing a medicament for treating or preventing a tumor, wherein the cancer is preferably breast cancer, ovarian cancer, cervical cancer, uterine cancer, prostate cancer, renal cancer, urethral cancer, bladder cancer, liver cancer, stomach cancer, endometrial cancer, salivary gland cancer, pancreatic cancer and lymphoma.

## INTERNATIONAL SEARCH REPORT

International application No.

**PCT/CN2021/096944**

**A. CLASSIFICATION OF SUBJECT MATTER**

A61K 31/357(2006.01)i; A61K 9/127(2006.01)i; A61K 47/16(2006.01)i; A61K 47/18(2006.01)i; A61K 47/24(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

DWPI, CNABS, CNTXT, CNKI, CNMED, baidu scholar: 艾日布林, 艾立布林, 乙二胺四乙酸, 磷酸, 喷替酸, 脂质体, 空白脂质体, 冻干, 柠檬酸, 包封率, 稳定性, eribulin, EDTA, phosphate, Pentetate Acid, liposome, blank, vacant, empty, Freeze-dried, citric acid, entrapment Efficiency, Stability

**C. DOCUMENTS CONSIDERED TO BE RELEVANT**

| Category* | Citation of document, with indication, where appropriate, of the relevant passages | Relevant to claim No. |
|---|---|---|
| Y | CN 102369008 A (EISAI R. & D. MANAGEMENT CO., LTD.) 07 March 2012 (2012-03-07) see abstract, claims 1-39, embodiments 1-6 | 1-30 |
| Y | CN 101601642 A (SHENYANG PHARMACEUTICAL UNIVERSITY) 16 December 2009 (2009-12-16) see abstract, claims 1-11, description page 2 paragraph 4 - page 9 paragraph 2, page 10 paragraph 3 from the bottom, page 13 paragraphs 2-3 from the bottom, page 15 paragraph 6 from the bottom - page 16 paragraph 2, experiment 1, tables 1-2, embodiment 1 | 1-30 |

☐ Further documents are listed in the continuation of Box C.　　☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **17 August 2021** | **31 August 2021** |

| Name and mailing address of the ISA/CN | Authorized officer |
|---|---|
| China National Intellectual Property Administration (ISA/CN)<br>No. 6, Xitucheng Road, Jimenqiao, Haidian District, Beijing 100088<br>China | |
| Facsimile No. **(86-10)62019451** | Telephone No. |

Form PCT/ISA/210 (second sheet) (January 2015)

| Patent document cited in search report | | | Publication date (day/month/year) | Patent family member(s) | | | Publication date (day/month/year) |
|---|---|---|---|---|---|---|---|
| CN | 102369008 | A | 07 March 2012 | CN | 102369008 | B | 29 October 2014 |
| | | | | HU | E029577 | T2 | 28 March 2017 |
| | | | | TW | 201102110 | A | 16 January 2011 |
| | | | | EP | 2415470 | A1 | 08 February 2012 |
| | | | | CO | 6430500 | A2 | 30 April 2012 |
| | | | | SI | EP2415470 | T1 | 30 December 2016 |
| | | | | AU | 2010232347 | A1 | 29 September 2011 |
| | | | | PL | 2415470 | T3 | 30 December 2016 |
| | | | | US | 2021023004 | A1 | 28 January 2021 |
| | | | | PE | 20120923 | A1 | 27 August 2012 |
| | | | | ZA | 201106535 | B | 30 May 2012 |
| | | | | ES | 2593027 | T3 | 05 December 2016 |
| | | | | TW | I392519 | B | 11 April 2013 |
| | | | | PE | 20151519 | A1 | 28 October 2015 |
| | | | | EP | 2415470 | A4 | 28 November 2012 |
| | | | | RU | 2476216 | C1 | 27 February 2013 |
| | | | | HK | 1165707 | A1 | 12 October 2012 |
| | | | | ME | 02518 | B | 20 February 2017 |
| | | | | BR | PI1014527 | A2 | 05 April 2016 |
| | | | | CA | 2756811 | A1 | 07 October 2010 |
| | | | | MX | 2011009632 | A | 19 October 2011 |
| | | | | IL | 215059 | A | 31 March 2016 |
| | | | | DK | 2415470 | T3 | 19 September 2016 |
| | | | | SM | T201600307 | B | 10 November 2016 |
| | | | | WO | 2010113984 | A1 | 07 October 2010 |
| | | | | US | 2014044777 | A1 | 13 February 2014 |
| | | | | RS | 55148 | B1 | 30 December 2016 |
| | | | | KR | 101495951 | B1 | 25 February 2015 |
| | | | | UA | 103794 | C2 | 25 November 2013 |
| | | | | EP | 2415470 | B1 | 06 July 2016 |
| | | | | MA | 33127 | B1 | 01 March 2012 |
| | | | | CY | 1118231 | T1 | 28 June 2017 |
| | | | | HR | P20161157 | T1 | 18 November 2016 |
| | | | | SG | 174255 | A1 | 28 October 2011 |
| | | | | US | 2012058178 | A1 | 08 March 2012 |
| | | | | JP | 5551683 | B2 | 16 July 2014 |
| | | | | KR | 20110122219 | A | 09 November 2011 |
| | | | | PT | 2415470 | T | 31 August 2016 |
| | | | | IL | 215059 | D0 | 30 November 2011 |
| | | | | CA | 2756811 | C | 23 September 2014 |
| | | | | SI | 2415470 | T1 | 30 December 2016 |
| | | | | BR | PI1014527 | B1 | 24 November 2020 |
| | | | | NZ | 595212 | A | 28 February 2014 |
| | | | | MY | 160203 | A | 28 February 2017 |
| | | | | LT | 2415470 | T | 10 October 2016 |
| | | | | JP | WO2010113984 | A1 | 11 October 2012 |
| CN | 101601642 | A | 16 December 2009 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010475697
- WO 2010113984 A
- CN 105163720